(19) **Europäisches Patentamt / European Patent Office / Office européen des brevets**

(11) **EP 2 133 083 A1**

(12) **EUROPEAN PATENT APPLICATION**
published in accordance with Art. 153(4) EPC

(43) Date of publication:
**16.12.2009 Bulletin 2009/51**

(21) Application number: **08722700.5**

(22) Date of filing: **24.03.2008**

(51) Int Cl.:
*A61K 33/20* $^{(2006.01)}$    *A61K 33/08* $^{(2006.01)}$
*A61P 1/00* $^{(2006.01)}$    *A61P 11/00* $^{(2006.01)}$
*A61P 15/00* $^{(2006.01)}$    *A61P 17/00* $^{(2006.01)}$
*A61P 27/00* $^{(2006.01)}$    *A61P 31/04* $^{(2006.01)}$
*A61P 31/10* $^{(2006.01)}$    *A61P 31/12* $^{(2006.01)}$

(86) International application number:
**PCT/JP2008/055384**

(87) International publication number:
**WO 2008/117767 (02.10.2008 Gazette 2008/40)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **27.03.2007 JP 2007082821**

(71) Applicant: **Taiko Pharmaceutical Co. Ltd.**
**Suita-shi**
**Osaka 564-0032 (JP)**

(72) Inventor: **SHIBATA, Takashi**
**Suita-shi**
**Osaka 564-0032 (JP)**

(74) Representative: **Lemcke, Brommer & Partner**
**Patentanwälte**
**Bismarckstraße 16**
**76133 Karlsruhe (DE)**

(54) **THERAPEUTIC AGENT FOR INFECTIOUS SKIN OR MUCOSAL DISEASE**

(57)    A therapeutic agent for infectious skin and mucosal diseases to be applied to an infected area for ameliorating a symptom of the infected area caused by infection with a pathogenic microorganism includes: a chlorine dioxide solution including a dissolved chlorine dioxide gas.

EP 2 133 083 A1

## Description

Technical field

The present invention relates to a therapeutic agent for infectious diseases of skin and mucosa (which means a therapeutic agent used for an infectious skin disease and an infectious mucosal disease. Hereinafter, also collectively referred to as "skin disease therapeutic agent") to be applied to an infected area for ameliorating a symptom of the infected area caused by infection with a pathogenic microorganism, and particularly to a skin disease therapeutic agent including dissolved chlorine dioxide, which can cure an intractable skin disease, such as skin disease caused by human papilloma virus, against which no effective therapeutic method had been developed.

Background art

Pathogenic microorganisms, such as human papilloma virus, that cause a skin disease or mucocutaneous disease through infection, enter a human body through minute scratches in skin or mucous, and cause wart in digits, or even cause condyloma acuminatum, various skin cancers or uterus cancer, and further may cause malignant tumor depending on the type of the virus. At present, a skin disease caused by human papilloma virus is treated, for example, by a pharmacotherapy in which a bleomycin ointment is applied, or an immunotherapy in which interferon is administered (see Patent Document 1).
As is known, the development of antibiotics, antimicrobial agents and antifungal agents advances, and many infectious skin diseases can be cured by the application of these agents. However, on the other hand, abuse of antibiotics or antimicrobial agents may result in the emergence of drug-resistant strains, and in the case of interferon also, the microorganisms may become refractory. Accordingly, cases in which a skin disease develops to be intractable are becoming notable.
Patent Document 1: published Japanese translation of a PCT application originally filed in English, No. 2005-525090

Disclosure of the invention

When considering a fact that substituted microbism and nosocomial infection became social problems, it is desirable to develop a novel therapeutic agent against a disease in human skin or skin mucosa which can be used as a substitute for the existing antibiotic or antimicrobial agent, antifungal agent and interferon.
The present invention is made with the view toward meeting such a demand, and the object is to provide a novel therapeutic agent for a skin disease caused by infection with a microorganism.
In a first aspect of the present invention for attaining the object above, there is provided a therapeutic agent for infectious skin and mucosal diseases to be applied to an infected area for ameliorating a symptom of the infected area caused by infection with a pathogenic microorganism, which includes: a chlorine dioxide solution including a dissolved chlorine dioxide gas.
In a second aspect of the present invention, there is provided a therapeutic agent for infectious skin and mucosal diseases to be applied to an infected area for ameliorating a symptom of the infected area caused by infection with a pathogenic microorganism, which includes: a chlorine dioxide solution including a dissolved chlorine dioxide gas, a chlorite, and a pH adjuster.
In a third aspect of the present invention, the chlorite is sodium chlorite, and the pH adjuster is an acid or a salt thereof having a buffering property whose pH is 2.5 to 6.8 as a 5% aqueous solution at 25°C.
In a fourth aspect of the present invention, the chlorite is sodium chlorite, and the pH adjuster is phosphoric acid or a salt thereof.
In a fifth aspect of the present invention, the chlorite is sodium chlorite, and the pH adjuster is sodium dihydrogenphosphate or a mixture of sodium dihydrogenphosphate with sodium monohydrogenphosphate.
In a sixth aspect of the present invention, the microorganism is bacterium.
In a seventh aspect of the present invention, the microorganism is fungus.
In an eighth aspect of the present invention, the microorganism is virus.
In a ninth aspect of the present invention, the virus is human papilloma virus.
In a tenth aspect of the present invention, the virus is adenovirus.
In an eleventh aspect of the present invention, the virus is herpesvirus.
The skin disease therapeutic agent of the present invention has an inactivation effect on a wide range of pathogenic microorganisms which cause a skin or mucosal disease, and at the same time, can ameliorate a skin disease caused by the microorganisms, and thus serves as a novel skin disease therapeutic agent which can be a substitute for the existing antibiotic, antimicrobial agent and antifungal agent.

Brief description of drawings

Fig. 1 is a photographic image showing a condition of an affected area on February 13th (at a first medical examination) during a cervicitis therapy in Example 3.

Fig. 2 is a photographic image showing a condition of the affected area on February 17th (under treatment) during the cervicitis therapy in Example 3.

Fig. 3 is a photographic image showing a condition of the affected area on February 19th (under treatment) during the cervicitis therapy in Example 3.

Fig. 4 is a photographic image showing a condition of the affected area on February 24th (under treatment) during the cervicitis therapy in Example 3.

Fig. 5 is a photographic image showing a condition of an affected area on January 19th (at a first medical examination) during a therapy of adenoma malignum of uterine cervix in Example 4.

Fig. 6 is a photographic image showing a condition of the affected area on February 26th (under treatment) during the therapy of adenoma malignum of uterine cervix in Example 4.

Fig. 7 shows graphs for coxsackievirus B5 inactivation regarding a skin disease therapeutic agent of the present invention ((a) shows a case where the sensitized time is 1 minute, and (b) shows a case where the concentration is 10ppm).

Best mode for carrying out the invention

The skin disease therapeutic agent of the present invention is a therapeutic agent for infectious skin and mucosal diseases to be applied to an infected area for ameliorating a symptom of the infected area caused by infection with a pathogenic microorganism, which includes: a chlorine dioxide solution containing a dissolved chlorine dioxide gas.

<Embodiment 1>

The skin disease therapeutic agent of the present embodiment includes a chlorine dioxide solution containing a dissolved chlorine dioxide gas, a chlorite and a pH adjuster. For example, the chlorite is sodium chlorite, the pH adjuster is an acid or a salt thereof having a buffering property whose pH is 2.5 to 6.8 as a 5% aqueous solution at 25°C. Further, the chlorite may be sodium chlorite, and the pH adjuster may be phosphoric acid or a salt thereof. Furthermore, the chlorite may be sodium chlorite, and the pH adjuster may be sodium dihydrogenphosphate or a mixture of sodium dihydrogenphosphate with sodium monohydrogenphosphate.

For the chlorite to be used in the present invention, for example, salts of alkali metal chlorite and salts of alkali earth metal chlorite can be mentioned. Examples of the salt of alkali metal chlorite include sodium chlorite, potassium chlorite and lithium chlorite. Examples of the salt of alkali earth metal chlorite include calcium chlorite, magnesium chlorite and barium chlorite. Especially, not only from the viewpoint of availability, but also from the viewpoint of excellent sustention of chlorine dioxide activity, sodium chlorite and potassium chlorite are preferable, and sodium chlorite is most preferable.

For the pH adjuster to be used in the present invention, any acid can be suitably used as long as it has a buffering property.

Examples of the organic acids and salts thereof include formic acid, acetic acid, propionic acid, butyric acid, lactic acid, pyruvic acid, citric acid, malic acid, tartaric acid, gluconic acid, glycolic acid, fumaric acid, malonic acid, maleic acid, oxalic acid, succinic acid, acrylic acid, crotonic acid, oxalic acid, glutaric acid, and salts thereof.

Examples of the inorganic acids include phosphoric acid, boric acid, metaphosphoric acid, pyrophosphoric acid, and sulfamic acid. Examples of the salts of the inorganic acid include salt (which may be sodium salt or potassium salt. The same applies to the descriptions hereinafter) of dihydrogenphosphate, and a mixture of salt of dihydrogenphosphate with salt of monohydrogenphosphate. It should be noted that one kind of the pH adjuster may be used alone or two or more kinds thereof may be used in combination.

From the viewpoint of excellent preservation stability and thus excellent antimicrobial activity after long-term preservation, an acid (inorganic acid and organic acid) or a salt thereof, having a buffering property whose pH is 2.5 to 6.8 as a 5% aqueous solution at 25°C, is preferable. When the pH is below 2.5, or above 6.8, the preservation stability of the dissolved chlorine dioxide is reduced, and a change in a liquid property (pH) of the chlorine dioxide solution during preservation becomes large.

It is preferable to use an acid (inorganic acid and organic acid) or a salt thereof having a buffering property whose pH is 3.5 to 6.0 as a 5% aqueous solution at 25°C, and it is more preferable to use one whose pH is 4.0 to 5.5. Among these, phosphoric acid or a salt thereof is preferably used as inorganic acid, and sodium dihydrogenphosphate and a mixture of sodium dihydrogenphosphate with sodium monohydrogenphosphate are more preferably used.

The chlorine dioxide solution may be, for example, obtained in the following manner. Specifically, (a) a chlorite is dissolved in water to prepare 2,000 to 180,000 ppm of an aqueous chlorite solution, (b) chlorine dioxide gas is bubbled and dissolved in water to prepare 100 to 2,900 ppm of an aqueous solution of chlorine dioxide, and (c) a chlorite is dissolved in water to prepare 2,000 to 180,000 ppm of an aqueous chlorite solution, and in the solution is dissolved a pH adjuster in an amount of 0.5 to 100 g per 1,000 ml of the solution, to prepare an aqueous chlorite solution containing the pH adjuster.

Subsequently, 5.0 to 990 ml, preferably 50 to 300 ml of the aqueous solution of chlorous acid (item (a)), 5.0 to 990 ml, preferably 50 to 800 ml of the aqueous solution of chlorine dioxide (item (b)) and 5.0 to 990 ml, preferably 50 to 400 ml of the aqueous solution of chlorous acid containing the pH adjuster (item (c)) are mixed and stirred well at room temperature to thereby prepare a chlorine dioxide solution.

It is preferred that the final pH of the chlorine dioxide solution is 4.5 to 6.5. When the pH is out of this range, the preservation stability is reduced, which may lead to, for example, change in the pharmacological activity during preservation, and to attenuation in the pharmacological activity after long-term (e.g. 2-year) preservation. In the present invention, more preferable pH range of the chlorine dioxide solution is 5.5 to 6.0.

<Embodiment 2>

The skin disease therapeutic agent of the present embodiment includes a chlorine dioxide solution including a chlorine dioxide gas dissolved therein, a chlorite and an acidic surfactant. For example, the chlorite is sodium chlorite, and the acidic surfactant is a mixed surfactant of sucrose fatty acid ester, sodium ctrate, propylene glycol and ethanol.

Instead of the pH adjuster, an acidic surfactant (an acid or a salt thereof having a buffering property whose pH is preferably 3.5 to 6.0, more preferaly 4.0 to 5.5 as a 5% aqueous solution at 25°C) may be used.

Examples of the acidic surfactants include, but are not restricted to, a phosphoric acid ester salt surfactant (such as polyoxyethylene phosphoric acid ester and a salt of alkyl phosphoric acid ester), a sulfonic acid salt surfactant (such as alkyl or alkylbenzene sulfonate, e.g. sodium lauryl sulfonate and sodium dodecylbenzenesulfonate; alkylnaphthalene sulfonate, e.g. sodium isopropylnaphthalene sulfonate; and alkyl diphenyl ether sulfonate), a sulfuric acid ester salt surfactant (such as alkyl or alkylbenzene sulfate, oxyethylene alkyl phenyl ether sulfate, and polyoxyethylene alkyl phenyl ether sulfate), and a carboxylic acid salt surfactant (such as alkyl sulfosuccinate). In addition, a mixed surfactant of sucrose fatty acid ester, sodium citrate, propylene glycol and ethanol, which is commercially available [Shokusen SE (manufactured by Mitsubishi-Kagaku Foods Corporation)], may be used.

<Embodiment 3>

The skin disease therapeutic agent of the present embodiment may include the chlorine dioxide solution including the chlorine dioxide gas dissolved therein, the chlorite and the pH adjuster, as well as a high water-absorbent resin (gel-like composition).

For example, the high water-absorbent resin is a starch-containing water-absorbent resin, a cellulose-containing water-absorbent resin or a synthetic polymer-containing water-absorbent resin; the chlorite is sodium chlorite; and the pH adjuster is sodium dihydrogenphosphate or a mixture of sodium dihydrogenphosphate with sodium monohydrogenphosphate.

The chlorine dioxide solution including the chlorine dioxide gas dissolved therein, the chlorite and the pH adjuster may be mixed with a high water-absorbent resin and prepared as a gel-like composition.

Examples of the high water-absorbent resin include a starch-containing water-absorbent resin (e.g., grafted starch-containing high water-absorbent resin, such as starch-acrylonitrile graft copolymer, starch-acrylic acid graft copolymer, starch-styrenesulfonic acid graft copolymer and starch-vinylsulfonic acid graft copolymer), a cellulose-containing water-absorbent resin (e.g., cellulose-containing high water-absorbent resin, such as cellulose-acrylonitrile graft copolymer, cellulose-styrenesulfonic acid graft copolymer, and cross-linked carboxymethyl cellulose; phosphoric-esterified paper and cloth; and carboxymethylated cloth), and a synthetic polymer-containing water-absorbent resin (e.g. polyvinyl alcohol-containing high water-absorbent resin, such as cross-linked polyvinyl alcohol; acrylic high water-absorbent resin, such as cross-linked polyacrylate, saponified polyacrylonitrile-containing polymer and cross-linked polyethylene glycol dimethacrylate; and cross-linked polyethylene oxide-containing high water-absorbent resin).

Examples of those commercially available include a starch/polyacrylic acid resin [Aqualic (powder, manufactured by Nippon Shokubai, Co., Ltd.), Sanwet (powder, manufactured by Sanyo Chemical Industries Ltd.)], a cross-linked polyacrylic acid resin [Arasorb (powder, manufactured by Arakawa Chemical Industries, Ltd.), Wondergel (powder, manufactured by Kao Corporation), Aqua Keep (powder, manufactured by Sumitomo Seika Chemicals, Co., Ltd.), Diawet (powder, manufactured by Mitsubishi Petrochemical Co., Ltd.)], an isobutylene/maleic acid resin [KI gel (powder, manufactured by Kuraray Co., Ltd.)], and a poval/polyacrylic acid salt resin [Sumikagel (powder, manufactured by Sumitomo Chemical Co., Ltd.)]. These may be used in implementing the present invention.

In the case where the gel-like composition is obtained by admixing with the high water-absorbent resin, for example, 50 to 99 weight % of the chlorine dioxide solution prepared in the manner as described in Embodiment 1 is added to 1.0 to 50 weight % of the high absorbent resin (powder), and stirred well at room temperature. Such a "gel-like composition" may be, for example, of general utility as being filled in a container having an opening on at least one side (see Japanese Patent Application JP61-40803A), or alternatively, of general utility as being filled in a container formed of paper or nonwoven fabric containing synthetic fiber as constituent fiber, with rims thereof sealed by heat-sealing the synthetic fiber or by a synthetic-resin adhesive. Examples of the synthetic fiber include the conventional thermoplastic synthetic fiber, such as polypropylene fiber, polyester fiber and polyamide fiber. In the case of the container formed of paper or nonwoven fabric containing such synthetic fiber as constituent fiber, it is possible to prevent clogging of the container which may otherwise be caused by the attached "gel-like composition", and at the same time to sustainably evaporate chlorine dioxide from the "gel-like composition".

<Embodiment 4>

The skin disease therapeutic agent of the present embodiment may include the chlorine dioxide solution including the chlorine dioxide gas dissolved therein and the chlorite, as well as an acidic high water-absorbent resin (gel-like composition).
For example, the acidic high water-absorbent resin is sodium salt cross-linkage in the acrylic acid polymerization, and the chlorite is sodium chlorite.

Instead of the pH adjuster, an acidic high water-absorbent resin (an acid or a salt thereof having a buffering property whose pH is preferaly 3.5 to 6.0, more preferably 4.0 to 5.5 as a 5% aqueous solution at 25°C) may be used. In this case, the dissolved chlorine dioxide gas and the chlorite are contained in the resin, which gives a gel-like composition.
Examples of the acidic high water-absorbent resin include, but are not restricted to, sodium salt cross-linkage in the acrylic acid polymerization [Aqualic (manufactured by Nippon Shokubai, Co., Ltd.)] commrecially available.

<Embodiment 5>

The skin disease therapeutic agent of the present embodiment may include the chlorine dioxide solution including the chlorine dioxide gas dissolved therein, the chlorite and the pH adjuster, as well as a foam agent (foaming composition).
For example, the foam agent may be formed of a surfactant and a foam stabilizer, or of a surfactant, a foam stabilizer and an aerosol propellant. In addition, for examle, the chlorite is sodium chlorite, the pH adjuster is sodium dihydrogenphosphate or a mixture of sodium dihydrogenphosphate with sodium monohydrogenphosphate.

The chlorine dioxide solution including the chlorine dioxide gas dissolved therein, the chlorite and the pH adjuster may be mixed with a foam agent, which gives a foaming composition.

The foam agent may be formed of (1) a surfactant and a foam stabilizer, or of (2) a surfactant, a foam stabilizer and an aerosol propellant.
Examples of the surfactant include, but are not restricted to, (1) at least one anionic surfactant selected from: a carboxylate salt, such as polyoxyethylene alkyl ether carboxylate; a sulfonate salt, such as alkylbenzenesulfonate and alkylnaphthalenesulfonate; a salt of sulfuric acid ester, such as salt of sulfuric acid higher alcohol ester; and a salt of phosphoric acid ester, such as polyoxyethylene alkyl ether phosphate, (2) a cationic surfactant, such as fatty acid quaternary ammonium salt, (3) a carboxybetaine type ampholytic surfactant, (4) a nonionic surfactant, such as polyoxyethylene alkyl ether, polyoxyethylene glycerin fatty acid ester, polyethylene glycol fatty acid ester, and fatty acid alkanolamide, (5) a fluorine-containing surfactant, and (6) a saponin.

Examples of the foam stabilizer include, but are not restricted to, (7) a stabilizer prepared by adding mono- or di-ethanolamine to the above-mentioned anionic surfactant, (8) a stabilizer prepared by adding a long-chain alcohol or alkylsulfoxide to the above-mentioned nonionic surfactant, and (9) liquid paraffin.

Examples of the aerosol propellant include, but are not restricted to, a high-pressure gas with low toxicity, such as liquefied natural gas (LPG), liquefied butane and dimethyl ether.

In the case where the foaming composition is prepared by adding the foam agent, the foaming composition may be prepared, for example, in a closed container, by adding 5.0 to 20 weight % of the foaming agent and 60 to 95 weight % of the surfactant to 1.0 to 20 weight % of the chlorine dioxide solution as prepared in Embodiment 1 above and stirring the mixture well at room temperature. Such a "foaming composition" may be enclosed in, for example, a trigger type foaming container, a pump type foaming container or the like.

<Skin disease (bacterial skin disease) to be treated>

Examples of the bacteria that cause bacterial skin disease include *Staphylococcus aureus, Pseudomonas aeruginosa, Streptococcus, Neisseria gonorrhoeae* and *Treponema pallidum,* and for treating the disease caused by these bacteria, such as those listed below, the skin disease therapeutic agent of the present invention can be used. Examples include acne vulgaris (acne), impetigo contagiosa, decubitus (bedsore), hordeolum (chalazion), blepharitis marginalis, periodental disease (such as alveolar pyorrhea), cellulitis, folliculitis, and staphylococcal scalded-skin syndrome. The agent of the present invention may be used for prevention of secondary infection, such as infection of injury, bum and surgical wound.

<Skin disease (fungal skin disease) to be treated>

Examples of the fungi that cause fungal skin disease include *Trichophyton, Malassezia* and *Candida,* and for treating the disease caused by these fungi, such as those listed below, the skin disease therapeutic agent of the present invention can be used. Examples include tinea pedis, tinea corporis, tinea cruris, tinea unguium, other Trichophyton infections, candidiasis (such as cutaneous candidiasis and vulval candidiasis), interdigital erosion, erythema blastomyceticum infantile, paronychia, tinea versicolor, and seborrheic dermatitis.

<Skin disease (viral skin disease) to be treated>

Examples of the viruses that cause viral skin disease include varicella-zoster virus, herpes simplex virus, adenovirus, enterovirus, human papilloma virus, pox virus, and coxsackievirus, and for treating the disease caused by these viruses, such as those listed below, the skin disease therapeutic agent of the present invention can be used. Examples include condyloma acuminatum, hand-foot-and-mouth disease, cervicitis, adenoma malignum of uterine cervix, pharyngeal papillomatosis, pharyngoconjunctival fever (pool fever), epidemic keratoconjunctivitis (pink-eye), acute hemorrhagic conjunctivitis, herpes labialis, herpes facialis, Kaposi varicelliform eruption, gluteal herpes, genital herpes, herpes whitlow, erythema multiforme exudativum, varicella, herpes zoster, oral mucositi, and stomatitis.

<Application region>

Examples of body regions to which the skin disease therapeutic agent of the present invention can be applied include scalp, face, eye (e.g., eyelid, conjunctiva or cornea), ear (e.g., external ear and external canal), nose (e.g., nostril and nasal mucosa), lip, oral cavity, pharynx, larynx, glottis, respiratory mucosa, esophagus, stomach, duodenum, small intestine, colon, tongue, gingiva (gum), neck, portion of torso, limbs (including interdigital area), penis, genital prepuce, vulva, vagina, uterine cervix, endometrium, anus, rectum, and nail. The skin disease therapeutic agent of the present invention is also suitably applied to mucous membrane.
In addition, it can be used for skin or skin mucosa, not only of human body, but also of pet, such as dog and cat, and domestic animal (such as cow, pig, chicken and sheep).

<Form of usage (dosage form) and amount used>

A preferable dosage form of the skin disease therapeutic agent of the present invention include, but is not restricted to, a solution, and a conventional substrate may be impregnated with the chlorine dioxide solution, to thereby prepare the material in a form of cream, gel, jelly, emulsion, paste, foam or the like (e.g., ointment, cream, lotion, propellant, liniment or the like), or in a form of cataplasm, plaster, tape or the like using the thus prepared material.
For such a substrate, any substrate can be used as long as it is pharmaceutically acceptable, and examples include: bees wax; fats, such as jojoba oil, olive oil, cacao oil, sesame oil, soybean oil, avocado oil, camellia oil, earthnut oil and polyoxyethylene-curing castor oil; mineral oils, such as white petrolatum, liquid paraffin, silicone oil, volatile silicone oil and petrolatum; higher fatty acids, such as lauric acid, myristic acid, stearic acid and oleic acid; lower alcohol, such as ethanol and isopropanol; triethanolamine and water.
Alternatively, a water-absorbing material is impregnated with the chlorine dioxide solution and applied to external ear, nostril, vagina or the like.

In addition, with respect to affected areas of mucous membrane inside the body, such as gastrointestinal mucosa (mucous membrane of stomach and intestines), endometrium and bronchial mucosa, the skin disease therapeutic agent of the present invention can be directly administered to the affected area using a known endoscope, a time-release capsule having the chlorine dioxide solution sealed therein, and the like.

Depending on its form, the skin disease therapeutic agent of the present invention can be prepared in accordance with conventional production methods (for example, common methods described in Pharmacopeia of Japan), and the thus produced skin disease therapeutic agent can be used on the affected area of skin by means of application, spraying, adhesion or insertion.

The use amount cannot be defined since it depends on the degree of symptom or size of the affected area, types of disease and the like, but in general, an appropriate amount of an agent having a chlorine dioxide concentration of 0.01 ppm to 500 ppm, preferably 0.1 ppm to 200 ppm, may be used once or 2 to 5 times per day.

[Example]

<Preparative Example (1) of chlorine dioxide solution>

In the following manner, a chlorine dioxide solution was prepared. Specifically, to 250 ml of water in which 2,000 ppm of chlorine dioxide gas had been dissolved were added 680 ml of water and 80 ml of a 25% solution of sodium chlorite, and stirred. Subsequently, to the solution was added sodium dihydrogenphosphate (whose pH is 4.1 to 4.5 as a 5% aqueous solution at 25°C) in such an amount that the pH of the solution became 5.5 to 6.0 and stirred, to thereby obtain 1,000 ml of a chlorine dioxide solution including chlorine dioxide gas dissolved therein, sodium chlorite, and sodium dihydrogenphosphate.

<Preparative example (2) of chlorine dioxide solution>

A chlorine dioxide solution was prepared in the same manner as in Preparative Example 1, except that citric acid (whose pH is 1.8 to 2.2 as a 5% aqueous solution at 25°C) as organic acid was used instead of sodium dihydrogenphosphate.

<Preservation stabilizing test>

The chlorine dioxide solutions obtaied in Preparative Examples 1 and 2 were diluted by a conventional method, to thereby prepare chlorine dioxide solutions having concentrations of 100 ppm and 500 ppm. In order to determine the preservation stability of these solutions, a change in the chlorine dioxide concentration (ppm) over time was measured. For the stabilizing test, an accelerated aging test (preservation temperature: 54°C, 14 days correspond to one year at normal temperature) was performed. The results of the preservation stability are shown in Tables 1 and 2 (comparative data is shown between a case where sodium dihydrogenphosphate was used in an amount of a reaction equivalent or more and a case where citric acid was used in an amount of a reaction equivalent or more).

[Table 1]

| *54°C-accelerated aging test (14 days correspond to one year at normal temperature) | | | | |
|---|---|---|---|---|
| (Concentration: 100 ppm, sodium dihydrogenphosphate was used) | | | (Concentration: 100 ppm, citric acid was used) | |
| | Dissolved $ClO_2$ (ppm) | | | Dissolved $ClO_2$ (ppm) |
| Start | 116 | | Start | 121 |
| 1 day later | 143 | | 1 day later | 1,551 |
| 5 days later | 154 | | 5 days later | 935 |
| 10 days later | 149 | | 10 days later | 269 |
| 15 days later | 128 | | 15 days later | 69 |
| 20 days later | 128 | | 20 days later | -- |
| 25 days later | 129 | | 25 days later | -- |

[Table 2]

| *54°C-accelerated aging test (14 days correspond to one year at normal temperature) | | | | | |
|---|---|---|---|---|---|
| (Concentration: 500 ppm, sodium dihydrogenphosphate was used) | | | (Concentration: 500 ppm, citric acid was used) | | |
| | Dissolved $ClO_2$ (ppm) | | | | Dissolved $ClO_2$ (ppm) |
| Start | 523 | | | Start | 567 |
| 1 day later | 554 | | | 1 day later | 3,474 |
| 5 days later | 546 | | | 5 days later | 1,160 |
| 10 days later | 532 | | | 10 days later | 286 |
| 15 days later | 538 | | | 15 days later | 150 |
| 20 days later | 516 | | | 20 days later | -- |
| 25 days later | 476 | | | 25 days later | -- |

As is apparent from Tables 1 and 2, sodium dihydrogenphosphate as the pH adjuster whose pH is 2.5 to 6.8 as a 5% aqueous solution at 25°C, remarkably enhances the preservation stability of the chlorine dioxide solution.

<Chlorine dioxide solution used in clinical study and antiviral test>

Chlorine dioxide solutions used in clinical study or antiviral test, which will be described later, were prepared by diluting them to various concentrations by a known method and leaving each of them in a container made of synthetic resin for 8 months. Specifically, the chlorine dioxide solutions obtained in Preparative Examples 1 and 2 were diluted and each dilution was introduced to a container made of synthetic resin having an opening with a diameter of 2 cm and left at room temperature for 8 months while the lid was closed.

[Example 1] Treatment of viral skin disease (condyloma acuminatum)

A penis of a male diagnosed as infected with condyloma acuminatum (causative virus: human papillomavirus), to whom the purpose of the present study was explained and who made an agreement, was treated under the supervision of a doctor. Specifically, to an affected area of the penis, an appropriate amount of the chlorine dioxide solution obtained in Preparative Example 1 (a chlorine dioxide concentration of 100 ppm as a dilution) was applied three times a day. One day after the application, white indurations in the affected area turned into red indurations. Two days later, the affected area became smaller, and three days later, turned into scab and eventually disappeared.

[Example 2] Treatment of viral skin disease (cervicitis)

A female who came to hospital with vaginal discharge as chief complaint (to whom the purpose of the present study was explained and who made an agreement) was examined by a doctor. The uterine cervix was erythrogenic and erosive, and small elevated lesion was partially obserbed. A viral test for human papilloma virus (HPV) (PCR normal value: 0 to 1 pg/ml) was also performed at the first medical examination (January 20th). Since the value was 301.59 pg/ml, the doctor diagnosed the disease as cervicitis by human papilloma virus (HPV).

A first treatment was given to this femal on January 25th. Specifically, a cloth (gauze) having a size of approximately 20 x 20 cm was impregnated with an appropriate amount of the chlorine dioxide solution obtained in Preparative Example 1 (concentration of diluted chlorine dioxide: 150 ppm), which was then shaped into a tampon, inserted into the vagina, and removed approximately 30 minutes later. Similar treatments were given on January 27th (second treatment) and January 30th (third treatment). As a result, redness of the uterine cervix disappeared, and a subjective symptom was in remission. In addition, a viral test for HPV was performed and the value was 0.41 pg/ml, which comfirms a remarkable therapeutic effect.

[Example 3] Treatment of viral skin disease (cervicitis)

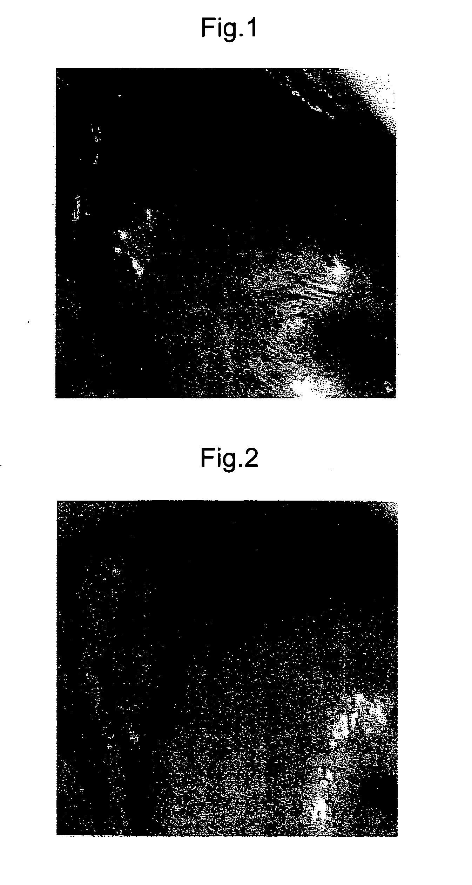

A female (age 25; to whom the purpose of the present study was explained and who made an agreement) was examined by a doctor. As a result, as shown in Fig. 1, a number of small elevated lesions each with mild erosion and redness having a size of 1 mm were obserbed in the uterine cervix and vaginal vestibule. A viral test for human papilloma virus (HPV) was also performed at the first medical examination (February 13th). Since the value was 33 pg/ml, the doctor diagnosed the disease as cervicitis by human papilloma virus.

A first treatment was given on the same day (February 13th). Specifically, a cloth (gauze) having a size of approximately 20 x 20 cm was impregnated with an appropriate amount of the chlorine dioxide solution obtained in Preparative Example 1 (concentration of diluted chlorine dioxide: 150 ppm), which was then shaped into a tampon, inserted into the vagina, and removed approximately 30 minutes later. Similar treatments were given on February 15th (second treatment) and February 17 (third treatment). Prior to the third treatment, the affected area was observed and it was confirmed that the lesions disappeared as shown in Fig. 2.

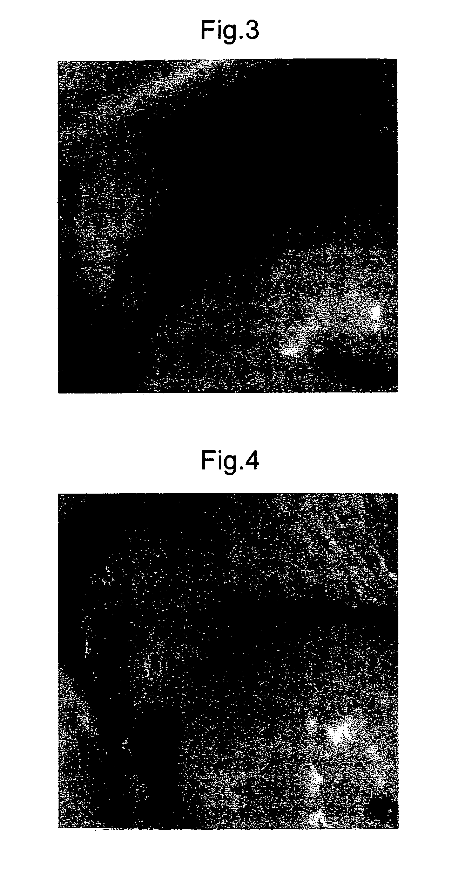

After three treatments in total, a viral test for HPV was performed on February 19th, and the value was 3.9 pg/ml. The resion at that time was partial roughness on the mucosal surface as shown in Fig. 3, and was in remission 5 days later (on February 24th) as shown in Fig. 4.

[Example 4] Treatment of viral skin disease (adenoma malignum of uterine cervix)

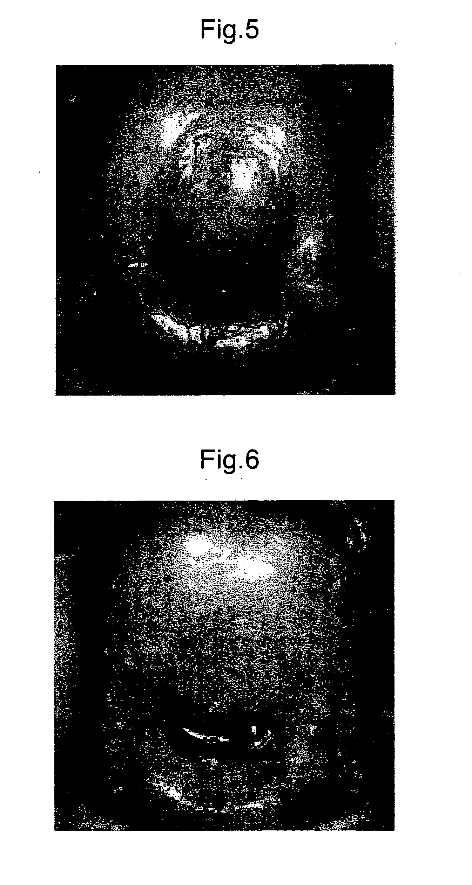

A female (age 45) who came to hospital was examined by a doctor using a colposcope. As a result, as shown in Fig. 5, erosion and hemorrhage were observed in the uterine cervix (January 19th). A viral test for HPV was also performed. Since the value was as high as 2,353.55 pg/ml, the doctor diagnosed the disease as a 1st stage of adenoma malignum of uterine cervix.

The attending doctor provided the female with a treatment by administering recombinant human interferon (rIFN) $\alpha$-2b vaginal effervescent capsules, but the treatment had no effect, and the female changed hospitals.

A doctor at a new hospital provided the female (to whom the purpose of the present study was explained and who made an agreement) with the following treatment three times by February 26th. Specifically, a cloth (gauze) having a size of approximately 20 x 20 cm was impregnated with an appropriate amount of the chlorine dioxide solution obtained in Preparative Example 1 (concentration of diluted chlorine dioxide: 150 ppm), which was then shaped into a tampon, inserted into the vagina, and removed approximately 30 minutes later.

As a result, as shown in Fig. 6, bleeding was stopped, enlargement of the uterine cervix was in remission, and a subjective symptom was improved. In addition, a viral test for HPV was performed and the value was remarkably reduced to 1,336.01 pg/ml.

[Example 5] Antiviral test (against coxsackievirus B5)

The chlorine dioxide solutions obtained in Preparative Examples 1 and 2 were added to coxsackievirus B5 (causative virus of hand-foot-and-mouth disease), and after a designated sensitized time elapsed, the agent was neutralized with a sodium thiosulfate solution, and a 10-fold dilution series was prepared. On the other hand, host cells (LLCMK2 cell) had been incubated at 37°C under 5%$CO_2$ for 3 days in a microplate (96 wells). The host cells were inoculated with the 10-fold dilution series, and cultured for 5 days. As a control, similar tests were performed using a stabilized aqueous solution of chlorine dioxide (aqueous solution of chlorite ion) (effective chlorine dioxide concentration: 10 ppm) and sodium hypochlorite (effective chlorine concentration: 10 ppm).

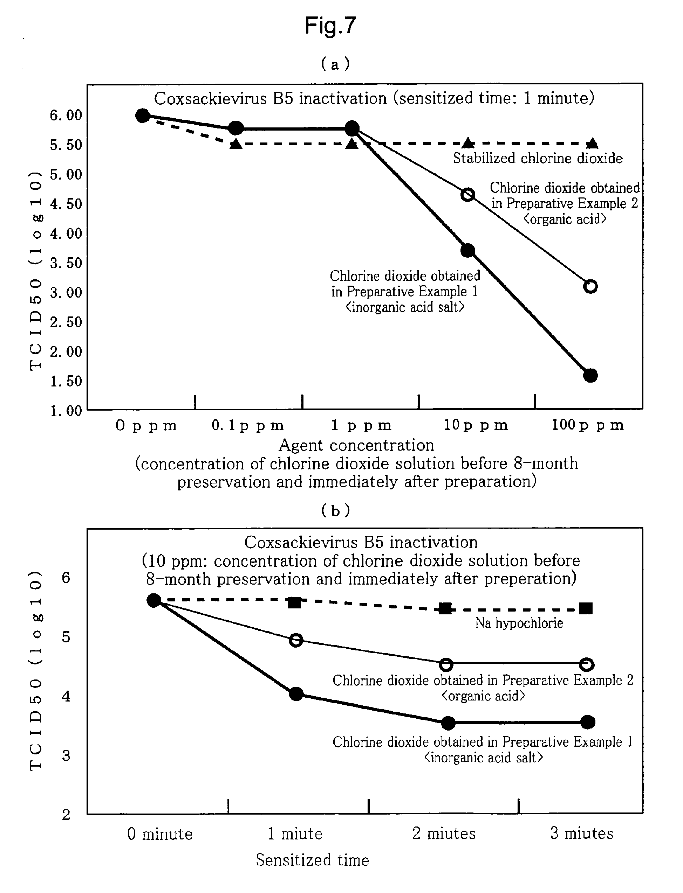

$TCID_{50}$ (50% Tissue Culture Infective Dose) ($\log 10$) was calculated using a cytopathogenic effect (CPE) by virus as a criterion, to thereby evaluate an antiviral activity effect of each chlorine dioxide solution. As a control, one diluted with distilled water, instead of the solution, was used. The results are shown in Figs. 7(a),(b). As is apparent from Fig. 7, the skin disease therapeutic agent of the present invention exhibited a remarkable inactivation effect against coxsackievirus B5 (hand-foot-and-mouth disease), and the agent at a concentration of 10 ppm with a sensitized time of 1 minute was 32 times, and of 2 minutes was 100 times, as effective as sodium hypochlorite.

[Example 6] Antifungal test (against *Trichophyton*)

To each of 20 patients (including females and males; to whom the purpose of the present study was explained and who made an agreement) diagnosed as tinea pedis (Trichophytia pompholyciformis), a doctor gave a spray container containing the chlorine dioxide solution obtained in Preparative Example 1 (concentration of diluted chlorine dioxide: 150 ppm), and instructed them to spray an appropriate amount thereof on the affected area of the foot, twice a day (at morning and night) at home. Two to three days after the initiation of the treatment, itchiness of the affected area disappeared in 15 patients out of 20, and one week later, the doctor's examination showed that vesiculation was disappeared from the affected area in 18 patients.

[Example 7] Antibacterial test (against *Staphylococcus*)

To each of 20 patients (including females and males; to whom the purpose of the present test was explained and who made an agreement) exhibiting pustular acne vulgaris caused by bacterial infection, a doctor gave a spray container containing the chlorine dioxide solution obtained in Preparative Example 1 (concentration of diluted chlorine dioxide: 150 ppm), and instructed them to impregnate a commercially available sterilized gauze with the solution and apply the gauze to the affected area of the face for approximately 3 minutes, twice a day (at morning and night) at home. One week later, the doctor's examination confirmed that the size of the pustula in the affected area became smaller in 17 patients out of 20, and no patients were newly pustulated.

[Example 8] Possibility of usage as therapeutic agent for adenoviral infection (1)

In the present Example, data will be shown that demostrates that the chlorine dioxide solution having a concentration of 10 ppm or more inactivates adenovirus that causes epidemic keratoconjunctivitis and the like, and that the chlorine dioxide solution having a concentration of 250 ppm does not cause ocular irritation.
Therefore, the chlorine dioxide solution having a concentration of at least 10 to 250 ppm may be effectively used as a therapeutic agent for adenoviral infection (epidemic keratoconjunctivitis) of bulbar conjunctiva in human. Since the solution can be effectively and safely used for an ocular mucosal disease, the solution can be also used as a therapeutic agent for a disease of at least mucosa portion to which the pharmacological stimulation becomes weaker than that to bulbar conjunctiva (such as oral cavity mucosa). The details will be described below.

(Anti-adenovirus test)

The method for the anti-adenovirus test will be described below.

    1. Test agent

       (1) The chlorine dioxide solution obtained in Preparative Example 1 (alternatively, Cleverin (registered trademark, manufactured by Taiko Pharmaceutical Co., Ltd., chlorine dioxide concentration: 500 ppm) may be used)
       (2) Sodium hypochlorite: manufactured by Nakarai Chemicals Ltd., highest quality; effective chlorine concentration (122,000 ppm)
       (3) Stabilized aqueous solution of chlorine dioxide (aqueous solution of chlorite ion) ("Splety" (product name) manufactured by Kindai-Chemical) (concentration: 4,988 ppm)

    2. Virus

Five-hundred ml of a culture medium of virus (canine adenovirus type 2 vaccine strain) which had been cultured with BK cell (bovine kidney primary cultured cell) was centrifuged at 3,000 rpm for 2 hours, and to the sediment was added 5 ml of distilled water to thereby prepare a viral suspension. The viral suspension was sterilized by filtration using a 0.45 $\mu$m membrane filter, and cryopreserved at -80°C until its usage.

    3. Adjustment of agent concentration

Each of the agents was diluted with sterile distilled water, to thereby prepare a dilution series of 1,250 ppm, 125 ppm, 12.5 ppm, 1.25 ppm, 0.125 ppm and 0.0125 ppm.

    4. Neutralizer

In order to neutralize chlorine dioxide in the agent, a 0.0025M sodium thiosulfate solution was prepared using Eagle MEM, and sterilized by filtration using a 0.22 $\mu$m membrane filter.

    5. Treatment of virus with agent

To each of the various concentrations of the agents (240 $\mu$l) was added virus (60 $\mu$l) and stirred, to thereby sensitize the virus with the agent, for 15, 30, 60, 120 or 180 seconds. Fourty $\mu$l of virus from each reaction liquid was added to a 0.0025M sodium thiosulfate solution (360 $\mu$l) to thereby neturalize chlorine dioxide. As a virus control, one diulted with distilled water, instead of the agent, was used.

    6. Dilution of agent-treated virus liquid

Each of the agent-treated virus liquid and the control virus liquid diluted with distilled water was neturalized with a 0.0025M sodium thiosulfate solution, and then neutralized virus (30 μl) was diluted with Eagle MEM (270 μl), to obtain a 10-fold dilution series of the treated virus.

7. Measurement of viral infectivity titer (TCID$_{50}$)

BK cell (50 μl/well), A549 cell (50 μl/well) and Vero cell (50 μl/well) were incubated in a 96-well microplate for 2 days, and the diluted virus was inoculated therein (50 μl/well, 4 well/dilution). The virus was allowed to be absorbed for 1 hour, and then 4% fetal bovine serum-added Eagle MEM was added to each well (50 μl/well). Each cell was cultured in a $CO_2$ incubator at 37°C for 5 to 8 days, and CPE was observed. TCID$_{50}$ was calculated based on Kerber's formula (equation 1).
[Equation 1]

$$logTCID_{50} = -L-d\,(S-0.5)$$

(L = minimum dilution ratio, d = dilution ratio, S = CPE prevalence for each dilution ratio)
The results are shown in Table 3. As is apparent from Table 3, adenovirus was deactivated with chlorine dioxide at a concentration of 10 ppm.
[Table 3]

| | | Antiviral activity of agent against canine adenovirus TCID$_{50}$(50 μL) | | | | | | |
|---|---|---|---|---|---|---|---|---|
| | Concentration | Sensitized time (second) | | | | | | |
| Agent | ppm | 0 | 15 | 30 | 60 | 120 | 180 | 240 |
| Chlorine dioxide (Preparative Example 1) | 100 | $10^{6.5}$ | $<10^{0.5}$ | $<10^{0.5}$ | $<10^{0.5}$ | $<10^{0.5}$ | $<10^{0.5}$ | $<10^{0.5}$ |
| | 10 | $10^{6.5}$ | $<10^{0.5}$ | $<10^{0.5}$ | $<10^{0.5}$ | $<10^{0.5}$ | $<10^{0.5}$ | $<10^{0.5}$ |
| | 1 | $10^{6.5}$ | $10^{6.0}$ | $10^{6.0}$ | $10^{6.0}$ | $10^{6.0}$ | $10^{6.0}$ | $10^{6.0}$ |
| | 0.1 | $10^{6.5}$ | $10^{6.0}$ | $10^{6.0}$ | $10^{6.0}$ | $10^{6.0}$ | $10^{6.0}$ | $10^{6.0}$ |
| | 0.01 | $10^{6.25}$ | $10^{6.0}$ | $10^{6.0}$ | $10^{6.0}$ | $10^{6.0}$ | $10^{6.0}$ | $10^{6.0}$ |
| Sodium hypochlorite | 1,000 | $10^{6.5}$ | $<10^{0.5}$ | $<10^{0.5}$ | $<10^{0.5}$ | $<10^{0.5}$ | $<10^{0.5}$ | $10^{0.5}$ |
| | 100 | $10^{6.5}$ | $<10^{0.5}$ | $<10^{0.5}$ | $<10^{0.5}$ | $<10^{0.5}$ | $<10^{0.5}$ | $<10^{0.5}$ |
| | 10 | $10^{6.5}$ | $10^{6.0}$ | $10^{6.0}$ | $10^{6.0}$ | $10^{6.0}$ | $10^{6.0}$ | $10^{6.0}$ |
| | 1 | $10^{6.5}$ | $10^{6.0}$ | $10^{6.0}$ | $10^{6.0}$ | $10^{6.0}$ | $10^{6.0}$ | $10^{6.0}$ |
| | 0.1 | $10^{6.25}$ | $10^{6.0}$ | $10^{6.0}$ | $10^{6.0}$ | $10^{6.0}$ | $10^{6.0}$ | $10^{6.0}$ |
| | 0.01 | $10^{6.25}$ | $10^{6.0}$ | $10^{6.0}$ | $10^{6.0}$ | $10^{6.0}$ | $10^{6.0}$ | $10^{6.0}$ |
| Stabilized aqueous solution of chlorine dioxide (aqueous solution of chlorite ion) | 1,000 | $10^{6.5}$ | $<10^{0.5}$ | $<10^{0.5}$ | $<10^{0.5}$ | $<10^{0.5}$ | $<10^{0.5}$ | $<10^{0.5}$ |
| | 100 | $10^{6.5}$ | $10^{6.0}$ | $10^{6.0}$ | $10^{6.0}$ | $10^{6.0}$ | $10^{6.0}$ | $10^{6.0}$ |
| | 10 | $10^{6.25}$ | $10^{6.0}$ | $10^{6.0}$ | $10^{6.0}$ | $10^{6.0}$ | $10^{6.0}$ | $10^{6.0}$ |
| | 1 | $10^{6.25}$ | $10^{6.0}$ | $10^{6.0}$ | $10^{6.0}$ | $10^{6.0}$ | $10^{6.0}$ | $10^{6.0}$ |
| | 0.1 | $10^{6.25}$ | $10^{6.0}$ | $10^{6.0}$ | $10^{6.0}$ | $10^{6.0}$ | $10^{6.0}$ | $10^{6.0}$ |
| | 0.01 | $10^{6.25}$ | $10^{6.0}$ | $10^{6.0}$ | $10^{6.0}$ | $10^{6.0}$ | $10^{6.0}$ | $10^{6.0}$ |

(Test regarding ocular irritation)

The method for the test regarding ocular irritation will be described below. 1. Test agent (test substance)
The chlorine dioxide solution obtained in Preparative Example 1 (chlorine dioxide concentration: 250 ppm)

2. Test system

(1) Animal

Eight male Japanese white rabbits (conventional) were purchased (weights at the time of the purchase were between 2.10 and 2.30 kg), and preliminarily reared for 8 days. From among these, 6 rabbits which are healthier, especially with no abnormality in eyes, were selected for the test.

(2) Identification

A stock number was written on the right ear of each rabbit with a black oil felt-tip pen at the time of the purchase, and for grouping, a test animal number was written on the left ear with a red oil felt-tip pen. In addition, each cage and a shelf for rearing was indicated with labels, and the animals were put in the cages and shelfs with the corresponding labels.

(3) Grouping

Upon grouping, it was visually confirmed on the day before the administration of the test substance that both eyes of the rabbits did not have abnormality. In addition, 0.1 ml of a 2% aqueous solution of sodium fluorescein were administered as eye drop and immediately thereafter the eyes were washed with 20 ml of injectable distilled water, and it was confirmed that no damage is present in the cornea using a slit lamp (manufactured by Neitz Instruments Co., Ltd.). In addition, on the day of the administration of the test substance, the weights of all animals were measured, and the healthy rabbits with the weight thereof falling within a range of 2.60 to 2.75 kg were selected and separated into two groups of unwashed-eye group and washed-eye group so that a difference in average weigths between two groups becomes small.

(4) Rearing management

Each of the amimals was separately kept in an aluminum blanket cage (width of 320 mm x length of 550 mm x height of 350 mm), and reared in a rabbit room (midium-sized animal room 2) under the following conditions: temperature of $22\pm3$ °C, humidity of $55\pm15\%$, ventilation of 10 or more times/hour, and artificial lighting of 8 hours/day (9 a.m. to 5 p.m.). Every day the animal room and rearing racks were cleaned, and thereafter, the floor was disinfected using NEO-CHLOR Clean (Shikoku Chemicals Corporation).
The animals were allowed to freely intake a commercially available solid feed RC4 (manufactured by Oriental Yeast Co., Ltd.) as feed stuff and tap water (water service managed by Chihayaakasaka village) as drinking water, the latter being served by autmoatic feed-water unit.

3. Test method

(1) Administration method

Six rabbits were separated into two groups each having three rabbits, in one of which group eyes were washed (washed-eye group), while in the other eyes were not washed (unwashed-eye group). For each animals, 0.1 ml of the test substance was administered once while keeping the lower eyelid of the right eye in a sack-like state, and in order to diffuse the test substance over the entire eyeball, the upper and lower eyelids were made closed for approximately 1 second. It should be noted that the left eye of each animal was used as untreated control. With respect to the administered eyes, the unwashed-eye group were left unwashed, and the washed-eye group were washed 1 minute later with 30 ml of injectable distilled water. Each animal of both groups was put in a collar made of stainless steel and held in a cage for 1 hour.

(2) Observation and measurement

During the observation period, general conditions of the animals were observed every day, and the weight was measured on the day of the administration of the test substance (day 0) and on the last day of the observation (7 days later). With respect to the right eye (the test substance was administred) and the left eye (untreated control) of the unwashed-eye group and washed-eye group, conditions of eyes were evaluated 1 hour, 24 hours, 72 hous, 4 days, and 7 days after the administration of the test substance by grading states of conjunctiva (edema, secretion and redness), cornea (level and area of opacification), and iris (degree), based on the evaluative criterion (Draize method) in Table 4.
[Table 4]

| Criterion for evaluating ocular mucosa irritancy (Draize method) | | |
|---|---|---|
| Region | Degree of reaction | Grade |
| A<br>Conjunctiva | (a) Edema<br>- No swelling<br>- Slight swelling than normal (including those in nictitating membrane)<br>- Apparent swelling, eyelid with a slight ectropion<br>- Swelling, eyelid half-closed<br>- Swelling, eyelid half-closed | 0<br>1<br>2<br>3<br>4 |
| | (b) Secretion<br>- No secretion<br>- Slightly larger amount than normal amount<br>- Presence of secretion, eyelid and hairs in contact with the eyelid are wet<br>- Presence of secretion, eyelid, hairs and considerable portions of areas around the eye are wet | 0<br>1<br>2<br>3 |
| | (c) Redness (eyelid conjunctiva and bulbar conjunctiva)<br>- Blood vessels are normal<br>- Apparent hyperemia of blood vessel as compared with normal condition -<br>Diffuse and crimson, individual blood vessel is not distinguishable<br>- Diffuse and beef-like red | 0<br>1<br>2<br>3 |
| B<br>Cornea | (a) Level of opacification (the densest area is used for evaluation)<br>- Transparent, no opacification<br>- Disseminated or diffuse opacification, iris is clearly distinguished<br>- Semi-transparent and easily distinguishable, details of iris is somewhat vague<br>- Opaque, details of iris is not distinguishable, size of the pupil is barely distinguishable<br>- White turbidity, iris is not recognized | 0<br>1<br>2<br>3<br>4 |
| | (b) Area of opacified portion in cornea<br>- 0 to 1/4<br>- 1/4 to 1/2<br>- 1/2 to 3/4<br>- 3/4 to 4/4 | 1<br>2<br>3<br>4 |
| C<br>Iris | (a) Degree<br>- Normal<br>- At least one of: plicae iridis larger than normal, hemostasis, swelling, and injection around cornea, is observed, but has reaction to light more or less.<br>- At least one of: no reaction to light, hemorrhage, and notable tissue disruption is observed | 0<br>1<br>2 |
| A: conjunctiva = (a + b + c) x 2 B: cornea = a x b x 5 C: iris = a x 5 | | |

4. Test result

During the observation period, no abnormality was observed in the general conditions of all rabbits, and the weights thereof steadily increased.
No distinctions were observed in comparison of increase in the average weight between the unwashed-eye group (test animals No. 1 to 3) and washed-eye group (test animals No. 4 to 6). When 0.1 ml of the test substance was administered once to the right eye of the rabbit, in the washed-eye group, 1 hour after the administration, all cases (3 cases) showed mild secretion and mild redness in conjunctiva, while eyelid edema was not recognized. These symptoms were alleviated over time, and disappeared 24 hours later in one case, and disappeared 48 hours later in the rest two cases. It should be noted that, the cornea and iris did not have abnormality during the observation period.

As for the results, the evaluations of the test animals No. 1 to 3 are shown in Tables 5 to 7, respectively, and the evaluations of the test animals No. 5 and 6 are shown in Tables 8 and 9, respectively. The comprehensive evaluation of the test animals No. 1 to 6 is shown in Table 10.

[Table 5]

| Evaluation of mucosal irritation in each rabbit, after single administration to right eye (Test animal No. 1: unwashed eye) | | | | | | | |
|---|---|---|---|---|---|---|---|
| Region | Observation item | Evaluation after test substance administration (right eye) | | | | | |
| | | 1 hr | 24 hrs | 48 hrs | 72 hrs | 4 days | 7 days |
| A Conjunctiva | a) Eyelid edema | 0 | 0 | 0 | 0 | 0 | 0 |
| | b) Secretion | 1 | 1 | 0 | 0 | 0 | 0 |
| | c) Redness | 1 | 1 | 0 | 0 | 0 | 0 |
| | (a+b+c)x2 | 4 | 4 | 0 | 0 | 0 | 0 |
| B Cornea | a) Opacification | 0 | 0 | 0 | 0 | 0 | 0 |
| | b) Opacified area | 0 | 0 | 0 | 0 | 0 | 0 |
| | axbx5 | 0 | 0 | 0 | 0 | 0 | 0 |
| C Iris | a) Degree of impairment | 0 | 0 | 0 | 0 | 0 | 0 |
| | a x 5 | 0 | 0 | 0 | 0 | 0 | 0 |
| Comprehensive evaluation grade A+B+C | | 4 | 4 | 0 | 0 | 0 | 0 |
| Note: The control (untreated) left eye had no abnormalities for all items | | | | | | | |

[Table 6]

| Evaluation of mucosal irritation in each rabbit, after single administration to right eye (Test animal No.2: unwashed eye) | | | | | | | |
|---|---|---|---|---|---|---|---|
| Region | Observation item | Evaluation after test substance administration (right eye) | | | | | |
| | | 1 hr | 24 hrs | 48 hrs | 72 hrs | 4 days | 7 days |
| A Conjunctiva | a) Eyelid edema | 0 | 0 | 0 | 0 | 0 | 0 |
| | b) Secretion | 1 | 1 | 0 | 0 | 0 | 0 |
| | c) Redness | 1 | 0 | 0 | 0 | 0 | 0 |
| | (a+b+c)x2 | 4 | 2 | 0 | 0 | 0 | 0 |
| B Cornea | a) Opacification | 0 | 0 | 0 | 0 | 0 | 0 |
| | b) Opacified area | 0 | 0 | 0 | 0 | 0 | 0 |
| | axbx5 | 0 | 0 | 0 | 0 | 0 | 0 |
| C Iris | a) Degree of impairment | 0 | 0 | 0 | 0 | 0 | 0 |
| | a x 5 | 0 | 0 | 0 | 0 | 0 | 0 |
| Comprehensive evaluation grade A+B+C | | 4 | 2 | 0 | 0 | 0 | 0 |
| Note: The control (untreated) left eye had no abnormalities for all items | | | | | | | |

[Table 7]

| Region | Observation item | Evaluation after test substance administration (right eye) | | | | | |
|---|---|---|---|---|---|---|---|
| Evaluation of mucosal irritation in each rabbit, after single administration to right eye (Test animal No. 3: unwashed eye) | | | | | | | |
| | | 1 hr | 24 hrs | 48 hrs | 72 hrs | 4 days | 7 days |
| A Conjunctiva | a) Eyelid edema | 0 | 0 | 0 | 0 | 0 | 0 |
| | b) Secretion | 1 | 0 | 0 | 0 | 0 | 0 |
| | c) Redness | 1 | 0 | 0 | 0 | 0 | 0 |
| | (a+b+c)x2 | 4 | 0 | 0 | 0 | 0 | 0 |
| B Cornea | a) Opacification | 0 | 0 | 0 | 0 | 0 | 0 |
| | b) Opacified area | 0 | 0 | 0 | 0 | 0 | 0 |
| | axbx5 | 0 | 0 | 0 | 0 | 0 | 0 |
| C Iris | a) Degree of impairment | 0 | 0 | 0 | 0 | 0 | 0 |
| | a x 5 | 0 | 0 | 0 | 0 | 0 | 0 |
| Comprehensive evaluation grade A+B+C | | 4 | 0 | 0 | 0 | 0 | 0 |
| Note: The control (untreated) left eye had no abnormalities for all items | | | | | | | |

[Table 8]

| Region | Observation item | Evaluation after test substance administration (right eye) | | | | | |
|---|---|---|---|---|---|---|---|
| Evaluation of mucosal irritation in each rabbit, after single administration to right eye (Test animal No. 5: washed eye) | | | | | | | |
| | | 1 hr | 24 hrs | 48 hrs | 72 hrs | 4 days | 7 days |
| A Conjunctiva | a) Eyelid edema | 0 | 0 | 0 | 0 | 0 | 0 |
| | b) Secretion | 0 | 0 | 0 | 0 | 0 | 0 |
| | c) Redness | 1 | 0 | 0 | 0 | 0 | 0 |
| | (a+b+c)x2 | 2 | 0 | 0 | 0 | 0 | 0 |
| B Cornea | a) Opacification | 0 | 0 | 0 | 0 | 0 | 0 |
| | b) Opacified area | 0 | 0 | 0 | 0 | 0 | 0 |
| | a x b x 5 | 0 | 0 | 0 | 0 | 0 | 0 |
| C Iris | a) Degree of impairment | 0 | 0 | 0 | 0 | 0 | 0 |
| | a x 5 | 0 | 0 | 0 | 0 | 0 | 0 |
| Comprehensive evaluation grade A+B+C | | 2 | 0 | 0 | 0 | 0 | 0 |
| Note: The control (untreated) left eye had no abnormalities for all items | | | | | | | |

[Table 9]

| Region | Observation item | Evaluation after test substance administration (right eye) | | | | | |
|---|---|---|---|---|---|---|---|
| Evaluation of mucosal irritation in each rabbit, after single administration to right eye (Test animal No. 6: washed eye) | | | | | | | |
| | | 1 hr | 24 hrs | 48 hrs | 72 hrs | 4 days | 7 days |
| A | a) Eyelid edema | 0 | 0 | 0 | 0 | 0 | 0 |

EP 2 133 083 A1

(continued)

| Evaluation of mucosal irritation in each rabbit, after single administration to right eye (Test animal No. 6: washed eye) | | | | | | | |
|---|---|---|---|---|---|---|---|
| Region | Observation item | Evaluation after test substance administration (right eye) | | | | | |
| | | 1 hr | 24 hrs | 48 hrs | 72 hrs | 4 days | 7 days |
| Conjunctiva | b) Secretion | 0 | 0 | 0 | 0 | 0 | 0 |
| | c) Redness | 1 | 0 | 0 | 0 | 0 | 0 |
| | (a+b+c)x2 | 2 | 0 | 0 | 0 | 0 | 0 |
| B Cornea | a) Opacification | 0 | 0 | 0 | 0 | 0 | 0 |
| | b) Opacified area | 0 | 0 | 0 | 0 | 0 | 0 |
| | a x b x 5 | 0 | 0 | 0 | 0 | 0 | 0 |
| C Iris | a) Degree of impairment | 0 | 0 | 0 | 0 | 0 | 0 |
| | a x5 | 0 | 0 | 0 | 0 | 0 | 0 |
| Comprehensive evaluation grade A+B+C | | 2 | 0 | 0 | 0 | 0 | 0 |
| Note: The control (untreated) left eye had no abnormalities for all items | | | | | | | |

[Table 10]

| Comprehensive evaluation of mucosal irritation test on rabbit, using the test substance | | | | | | | |
|---|---|---|---|---|---|---|---|
| Test group | Test animal No. | Comprehensive evaluation after administration (right eye) | | | | | |
| | | 1 hr | 24 hrs | 48 hrs | 72 hrs | 4 days | 7 days |
| Unwashed eye | 1 | 4 | 4 | 0 | 0 | 0 | 0 |
| | 2 | 4 | 2 | 0 | 0 | 0 | 0 |
| | 3 | 4 | 0 | 0 | 0 | 0 | 0 |
| Average | | 4.0 | 2.0 | 0.0 | 0.0 | 0.0 | 0.0 |
| Washed eye | 4 | 2 | 0 | 0 | 0 | 0 | 0 |
| | 5 | 2 | 0 | 0 | 0 | 0 | 0 |
| | 6 | 2 | 0 | 0 | 0 | 0 | 0 |
| Average | | 2.0 | 0.0 | 0.0 | 0.0 | 0.0 | 0.0 |
| Note: The control (untreated) left eye had no abnormalities for all items | | | | | | | |

[Example 9] Possibility of usage as therapeutic agent for adenoviral infection (2)

Substantially the same test as in Example 8 was performed, except that the human adenovirus type 2 was used instead of the canine adenovirus type 2. The antiviral activity of each agent is shown in Table 11.

[Table 11]

| | Antiviral activity of agent against human adenovirus $TCID_{50}$(50 $\mu$L) | | | | | | | |
| | Concentration | Sensitized time (second) | | | | | | |
| Agent | ppm | 0 | 15 | 30 | 60 | 120 | 180 | 240 |
| Chlorine dioxide (Preparative Example 1) | 100 | $10^{6.5}$ | $<10^{0.5}$ | $<10^{0.5}$ | $<10^{0.5}$ | $<10^{0.5}$ | $<10^{0.5}$ | $<10^{0.5}$ |
| | 10 | $10^{6.5}$ | $10^{1.5}$ | $10^{1.5}$ | $10^{1.5}$ | $10^{1.5}$ | $10^{1.5}$ | $10^{1.5}$ |
| | 1 | $10^{6.5}$ | $10^{5.0}$ | $10^{5.0}$ | $10^{5.0}$ | $10^{5.0}$ | $10^{5.0}$ | $10^{5.0}$ |
| | 0.1 | $10^{6.5}$ | $10^{6.0}$ | $10^{6.0}$ | $10^{6.0}$ | $10^{6.0}$ | $10^{6.0}$ | $10^{6.0}$ |
| Sodium hypo-chlorite | 1,000 | $10^{6.5}$ | $<10^{0.5}$ | $<10^{0.5}$ | $<10^{0.5}$ | $<10^{0.5}$ | $<10^{0.5}$ | $10^{0.5}$ |
| | 100 | $10^{6.5}$ | $10^{1.5}$ | $10^{1.5}$ | $10^{1.5}$ | $10^{1.5}$ | $10^{1.5}$ | $10^{1.5}$ |
| | 10 | $10^{6.5}$ | $10^{5.5}$ | $10^{5.5}$ | $10^{5.5}$ | $10^{5.5}$ | $10^{5.5}$ | $10^{5.5}$ |
| | 1 | $10^{6.5}$ | $10^{6.0}$ | $10^{6.0}$ | $10^{6.0}$ | $10^{6.0}$ | $10^{6.0}$ | $10^{6.0}$ |
| Stabilized aqueous solution of chlorine dioxide | 1,000 | $10^{3.5}$ | $10^{3.5}$ | $10^{3.5}$ | $10^{3.5}$ | $10^{3.5}$ | $10^{3.5}$ | $10^{3.5}$ |
| | 100 | $10^{6.5}$ | $10^{6.0}$ | $10^{6.0}$ | $10^{6.0}$ | $10^{6.0}$ | $10^{6.0}$ | $10^{6.0}$ |
| | 10 | $10^{6.5}$ | $10^{6.0}$ | $10^{6.0}$ | $10^{6.0}$ | $10^{6.0}$ | $10^{6.0}$ | $10^{6.0}$ |
| | 1 | $10^{6.5}$ | $10^{6.0}$ | $10^{6.0}$ | $10^{6.0}$ | $10^{6.0}$ | $10^{6.0}$ | $10^{6.0}$ |

(Conclusion)

The test substance was administred once to the right eye of each of six rabbits, and the rabbits were separated into two groups, in one of which group (3 rabbits) the right eye was washed with injectable distilled water one minute after the administration, while in the other group (3 rabbits) the right eye was not washed, and ocular irritation caused by the test substance was observed for 7 days. The left eye was used as untreated control. In the unwashed-eye group, 1 hour after the administration, all cases showed mild secretion and mild redness in conjunctiva. These symptoms were alleviated over time, and disappeared 48 hours later in all cases. On the other hand, in the washed-eye group, 1 hour after the administration, all cases showed mild redness in conjunctiva, and they disappeared 24 hours later in all cases. In both groups, no abnormalities were observed in cornea and iris. In this manner, ocular irritation of the test substance (chlorine dioxide solution) was alleviated by washing the eye.

As is apparent from the data shown above, it was elucidated that the chlorine dioxide solution having a concentration of at least 10 to 250 ppm may be effectively used as a therapeutic agent for adenoviral infection (epidemic keratoconjunctivitis) of bulbar conjunctiva in human. Since the solution can be effectively and safely used as a therapeutic agent for an ocular mucosal disease, the solution can be also used as a therapeutic agent for a disease of at least mucosa portion to which the pharmacological stimulation becomes weaker than that to bulbar conjunctiva (such as oral cavity mucosa).

[Example 10] Antiviral activity against herpesvirus type 1

An antiviral activity against herpesvirus type 1 (HSV-1, Herpes simplex virus type 1) (virus which causes mainly herpes labialis, and also causes herpetic stomatitis, herpetic keratitis and herpes simplex encephalitis) was measured. The method will be described below.

1. Test agent

(1) The chlorine dioxide solution obtained in Preparative Example 1 (alternatively, Cleverin (registered trademark, manufactured by Taiko Pharmaceutical Co., Ltd., chlorine dioxide concentration: 500 ppm) may be used)
(2) Sodium hypochlorite: manufactured by Nakarai Chemicals Ltd., highest quality; effective chlorine concentration (122,000 ppm)
(3) Stabilized aqueous solution of chlorine dioxide (aqueous solution of chlorite ion) ("Splety" (product name) manufactured by Kindai-Chemical) (concentration: 4,988 ppm)

2. Virus

Five-hundret ml of a culture solution of virus (herpesvirus type 1 kos) which had been cultured with CRFK cell (feline kidney cell) was centrifuged at 3,000 rpm for 3 hours, and to the sediment was added 4 ml of distilled water to thereby prepare a viral suspension. The viral suspension was sterilized by filtration using a 0.45 $\mu$m membrane filter, and cryopreserved at -80°C until its usage.

3. Adjustment of agent concentration

Each of the agents was diluted with sterile distilled water, to thereby prepare a dilution series of 1,250 ppm, 125 ppm, 12.5 ppm, 1.25 ppm and 0.125 ppm.

4. Neutralizer

In order to neutralize chlorine dioxide in the agent, a 0.0025M sodium thiosulfate solution was prepared using Eagle MEM, and sterilized by filtration using a 0.22 $\mu$m membrane filter.

5. Treatment of virus with agent

To each of the various concentrations of the agents (240 $\mu$l) was added virus (60 $\mu$l) and stirred, to thereby sensitize the virus with the agent, for 15, 30, 60, 120 or 180 seconds. Fourty $\mu$l of virus from each reaction liquid was added to a 0.0025M sodium thiosulfate solution (360$\mu$l), to thereby neutralize chlorine dioxide. As a virus control, one diulted with distilled water, instead of the agent, was used.

6. Dilution of agent-treated virus liquid

Each of the agent-treated virus liquid and the control virus liquid diluted with distilled water was neutralized with a 0.0025M sodium thiosulfate solution, and then neutralized virus (100 $\mu$l) was diluted with Eagle MEM (900 $\mu$l), to obtain a 10-fold dilution series of the treated virus.

7. Measurement of viral infectivity titer ($TCID_{50}$)

CRFK cell (50 $\mu$l/well) was incubated in a 96-well microplate for 3 days, and the diluted virus was inoculated therein (50 $\mu$l/well, 4 well/dilution). Each cell was cultured in a $CO_2$ incubator at 37°C for 5 days, and CPE was observed. $TCID_{50}$ was calculated based on the above-mentioned equation 1 (Kerber's formula).
The results are shown in Table 12.
[Table 12]

| Antiviral activity of each agent against herpesvirus type 1 (HSV1 kos) | | | | | | |
|---|---|---|---|---|---|---|
| | | Sensitized time (second) | | | | |
| | Sensitizing concentration | 15 | 30 | 60 | 120 | 180 |
| Chlorine dioxide (Preparative Example 1) | 10 ppm | $10^{1.50}$ | $<10^{0.5}$ | $<10^{0.5}$ | $<10^{0.5}$ | $<10^{0.5}$ |
| | 1 ppm | $10^{3.50}$ | $10^{3.50}$ | $10^{3.00}$ | $10^{3.00}$ | $10^{3.00}$ |
| | 0.1 ppm | $10^{4.25}$ | $10^{4.00}$ | $10^{3.75}$ | $10^{3.75}$ | $10^{3.75}$ |
| | 0.01 ppm | $10^{4.75}$ | $10^{4.50}$ | $10^{4.25}$ | $10^{4.25}$ | $10^{3.00}$ |
| Sodium hypochlorite | 10 ppm | $10^{3.75}$ | $10^{3.75}$ | $10^{3.25}$ | $10^{3.00}$ | $10^{4.25}$ |
| | 1 ppm | $10^{4.75}$ | $10^{4.50}$ | $10^{4.25}$ | $10^{4.25}$ | $10^{4.25}$ |
| | 0.1 ppm | $10^{4.50}$ | $10^{4.00}$ | $10^{4.25}$ | $10^{4.25}$ | $10^{4.25}$ |
| | 0.01 ppm | $10^{5.00}$ | $10^{4.50}$ | $10^{4.25}$ | $10^{4.25}$ | $10^{4.25}$ |

(continued)

| Antiviral activity of each agent against herpesvirus type 1 (HSV1 kos) | | | | | | |
|---|---|---|---|---|---|---|
| | | Sensitized time (second) | | | | |
| | Sensitizing concentration | 15 | 30 | 60 | 120 | 180 |
| Stabilized aqueous solution of chlorine dioxide (aqueous solution of chlorite ion) | 10 ppm | $10^{4.25}$ | $10^{4.25}$ | $10^{4.25}$ | $10^{4.25}$ | $10^{4.25}$ |
| | 1 ppm | $10^{4.25}$ | $10^{4.25}$ | $10^{4.25}$ | $10^{4.25}$ | $10^{4.25}$ |
| | 0.1 ppm | $10^{4.25}$ | $10^{4.25}$ | $10^{4.25}$ | $10^{4.25}$ | $10^{4.25}$ |
| | 0.01 ppm | $10^{4.25}$ | $10^{4.25}$ | $10^{4.25}$ | $10^{4.25}$ | $10^{4.25}$ |
| Virus control | 0 ppm | $10^{5.00}$ | (Sensitized time 0 second) | | | |

(Result)

1. Antiviral activity of chlorine dioxide against herpesvirus type 1

The purified herpesvirus type 1 was sensitized with various concentrations of chlorine dioxide (final concentration of 0.01 to 10 ppm) for 15, 30, 60, 120 or 180 seconds, and then viral infectivity titer was measured, to thereby measure antiviral activity of chlorine dioxide against herpesvirus type 1. When the virus was sensitized with 10 ppm of chlorine dioxide, the viral infectivity titer was reduced by 3.5log with the sensitizing period of 15 seconds or lesss, and by 4.5log or more with 30 seconds or more. However, in the case of 1 ppm, the titer was reduced by 1.5log with 30 seconds, and by 2log even with 180 seconds.

2. Antiviral activity of sodium hypochlorite against herpesvirus type 1

When the viurs was sensitized with 10 ppm of sodium hypochlorite, the virus was not likely to be inactivated as compared with chlorine dioxide, and the infectivity titer was reduced by 1log or less even with the sensitizing period of 180 seconds. When sensitized with 1 ppm or less, apparent reduction of the infectivity titer was not observed.
3. Antiviral activity of stabilized aqueous solution of chlorine dioxide (aqueous solution of chlorite ion) against herpesvirus type 1
When the virus was sensitized with 10 ppm of the stabilized aqueous solution of chlorine dioxide for 180 seconds, reduction of the viral infectivity titer was not observed.

(Discussion)

The antiviral activity of the chlorine dioxide-containing disinfectant, such as chlorine dioxide, sodium hypochlorite and the stabilized aqueous solution of chlorine dioxide (aqueous solution of chlorite ion), against herpesvirus type 1 [large virus having DNA genes enclosed in an envelope] were measured.
Herpesvirus type 1 is an important virus as causative virus for herpes labialis, herpetic stomatitis, herpetic keratitis, Kaposi varicelliform eruption, herpes encephalitis or the like.
As a result, when the virus is sensitized with chlorine dioxide having a concentration of 10 ppm for 15 seconds, the infectivity titer was reduced by 3.0log or more. On the other hand, in the case of sodium hypochlorite and the stabilized aqueous solution of chlorine dioxide, apparent reduction of the infectivity titer was not observed.

[Example 11] Antiviral activity against herpesvirus type 2

The antiviral activity against herpesvirus type 2 (HSV-2, Herpes simplex virus type 2) (virus which causes mainly genital herpes, neonatal herpes, herpetic meningitis and herpetic myelitis) was measured. Substantially the same test as in Example 10 was performed, except that the herpesvirus type 2 (standard strain; UF strain) was used instead of the herpesvirus type 1. The results are shown in Table 13.
[Table 13]

| Antiviral activity of each agent against herpesvirus type 2 (scientific name: HHV2) (HSV2 UF-strain) | $TCID_{50}$/50 μL unit ($10^n$) | | | |
|---|---|---|---|---|
| Agent | Agent sensitized time (second) | | | |
| | 0 | 30 | 60 | 120 |
| Control (purified water) | 4.25 | 4.25 | 4.25 | 4.25 |
| Chlorine dioxide (Preparative Example 1), 10 ppm | 4.25 | 0 | 0 | 0 |
| Chlorine dioxide (Preparative Example 1), 1 ppm | 4.25 | 4 | 3.75 | 3.5 |
| Chlorine dioxide (Preparative Example 1), 0.1 ppm | 4.25 | 4.25 | 4.25 | 4.25 |
| Sodium hypochlorite, 10 ppm | 4.25 | 0 | 0 | 0 |
| Sodium hypochlorite, 1 ppm | 4.25 | 4 | 3.75 | 3.75 |
| Sodium hypochlorite, 0.1 ppm | 4.25 | 4.25 | 4.25 | 4.25 |
| Stabilized aqueous solution of chlorine dioxide (aqueous solution of chlorite ion), 10ppm | 4.25 | 3.75 | 3.75 | 3.75 |
| Stabilized aqueous solution of chlorine dioxide (aqueous solution of chlorite ion), 1ppm | 4.25 | 4 | 3.75 | 3.75 |
| Stabilized aqueous solution of chlorine dioxide (aqueous solution of chlorite ion), 0.1ppm | 4.25 | 4.25 | 4.25 | 4.25 |

(Overview)

It was elucidated that the skin disease therapeutic agent of the present invention has an effect on skin diseases caused by a wide range of pathogenic microorganisms, even after a long-term preservation. In addition, the agent is effective at low concentrations, and thus have low irritancy. Adverse effect on human body, especially on human mucosa, can be suppressed to a minimum and accordingly, the agent can be widely applied in a clinical field as a skin disease therapeutic agent.

Furthermore, the followings can be noted. Specifically, in the skin disease therapeutic agent of the present invention, excellent preservation stability can be obtained. For example, the concentration of the dissolved chlorine dioxide can be maintained constant for a long term, that is, even when chlorine dioxide is continuously released by portions as gas from the skin disease therapeutic agent, the chlorine dioxide concentration in the skin disease therapeutic agent can be held in an approximately constant range. The expression "continuously released by portions as gas" herein means that, for example, during transportation or preservation, even though a lid of a container is closed, chlorine dioxide dissipates as gas in the course of nature.

When phosphoric acid or salt thereof, preferably sodium dihydrogenphosphate or a mixture of sodium dihydrogenphosphate with disodium hydrogenphosphate, is used as an inorganic acid or a salt thereof having a buffering property (pH adjuster), as compared with other inorganic acids (or salt thereof) or organic acids, the preservation stability is further improved (period with the preservation stability is further extended), and fluctuation (change) in a liquid property (pH) over time during preservation is suppressed. Accordingly, when the skin disease therapeutic agent of the present invention is preserved for a long term (e.g., 1 year to 3 years), it is preferred that phosphoric acid or a salt thereof, preferably sodium dihydrogenphosphate or a mixture of sodium dihydrogenphosphate with disodium hydrogenphosphate is used as the pH adjuster, and that they are combined with sodium chlorite and used as a composition. By selecting sodium dihydrogenphosphate or the mixture of sodium dihydrogenphosphate with disodium hydrogenphosphate, and by combining this with sodium chlorite, the preservation stability is further improved, and fluctuation in the chlorine dioxide concentration over time during preservation is further suppressed. In other words, an excessive progression of a reaction in which sodium chlorite turns into chlorine dioxide hardly occurs, and thus a gas equilibration state is retained by replenishing chlorite ion from sodium chlorite that compensates only chlorine dioxide that is lost by natural decomposition or that dissipates from a lid portion or walls of the container.

As described above, unnecessary consumption of sodium chlorite is suppressed and sodium chlorite is efficiently consumed, leading to further improvement in the preservation stability (period with the preservation stability is further extended), and to further suppression of fluctuation (change) in the chlorine dioxide concentration over time during preservation (both the decrease and increase in the concentration can be suppressed).

In addition, a mechanism of the skin disease therapeutic agent for replenishing chlorine dioxide from sodium chlorite for a long term is exerted, even on skin and skin mucosa to which the skin disease therapeutic agent is applied. This provides an excellent sustained effect to the patient, i.e. lasting inactivation action against pathogenicity of the skin disease therapeutic agent, and thus more effective treatment of skin diseases.

Industrial applicability

The present invention can be used as a therapeutic agent for infectious skin and mucosal diseases to be applied to an infected area, for ameliorating a symptom of the infected area caused by infection with a pathogenic microorganism.

**Claims**

1.  An therapeutic agent for infectious skin and mucosal diseases to be applied to an infected area for ameliorating a symptom of the infected area caused by infection with a pathogenic microorganism, which comprises:

    a chlorine dioxide solution comprising a dissolved chlorine dioxide gas.

2.  A therapeutic agent for infectious skin and mucosal diseases to be applied to an infected area for ameliorating a symptom of the infected area caused by infection with a pathogenic microorganism, which comprises:

    a chlorine dioxide solution comprising a dissolved chlorine dioxide gas, a chlorite, and a pH adjuster.

3.  The therapeutic agent for infectious skin and mucosal diseases according to Claim 2, wherein
    the chlorite is sodium chlorite, and
    the pH adjuster is an acid or a salt thereof having a buffering property whose pH is 2.5 to 6.8 as a 5% aqueous solution at 25°C.

4.  The therapeutic agent for infectious skin and mucosal diseases according to Claim 2, wherein
    the chlorite is sodium chlorite, and
    the pH adjuster is phosphoric acid or a salt thereof.

5.  The therapeutic agent for infectious skin and mucosal diseases according to Claim 2, wherein
    the chlorite is sodium chlorite, and
    the pH adjuster is one of sodium dihydrogenphosphate and a mixture of sodium dihydrogenphosphate with sodium monohydrogenphosphate.

6.  The therapeutic agent for infectious skin and mucosal diseases according to any one of Claims 1 to 5, wherein the microorganism is bacterium.

7.  The therapeutic agent for infectious skin and mucosal diseases according to any one of Claims 1 to 5, wherein the microorganism is fungus.

8.  The therapeutic agent for infectious skin and mucosal diseases according to any one of Claims 1 to 5, wherein the microorganism is virus.

9.  The therapeutic agent for infectious skin and mucosal diseases according to Claim 8, wherein the virus is human papilloma virus.

10. The therapeutic agent for infectious skin and mucosal diseases according to Claim 8, wherein the virus is adenovirus.

11. The therapeutic agent for infectious skin and mucosal diseases according to Claim 8, wherein the virus is herpesvirus.

Fig.1

Fig.2

## Fig.3

## Fig.4

Fig.5

Fig.6

## Fig.7

(a)

Coxsackievirus B5 inactivation (sensitized time: 1 minute)

Stabilized chlorine dioxide

Chlorine dioxide obtained in Preparative Example 2 〈organic acid〉

Chlorine dioxide obtained in Preparative Example 1 〈inorganic acid salt〉

TCID50 (log10)

Agent concentration
(concentration of chlorine dioxide solution before 8-month preservation and immediately after preparation)

(b)

Coxsackievirus B5 inactivation
(10 ppm: concentration of chlorine dioxide solution before 8-month preservation and immediately after preperation)

Na hypochlorie

Chlorine dioxide obtained in Preparative Example 2 〈organic acid〉

Chlorine dioxide obtained in Preparative Example 1 〈inorganic acid salt〉

TCID50 (log10)

Sensitized time

<div align="center">**INTERNATIONAL SEARCH REPORT**</div>

| International application No. |
|---|
| PCT/JP2008/055384 |

**A. CLASSIFICATION OF SUBJECT MATTER**
*A61K33/20*(2006.01)i, *A61K33/08*(2006.01)i, *A61P1/00*(2006.01)i, *A61P11/00*
(2006.01)i, *A61P15/00*(2006.01)i, *A61P17/00*(2006.01)i, *A61P27/00*(2006.01)i,
*A61P31/04*(2006.01)i, *A61P31/10*(2006.01)i, *A61P31/12*(2006.01)i
According to International Patent Classification (IPC) or to both national classification and IPC

**B. FIELDS SEARCHED**

Minimum documentation searched (classification system followed by classification symbols)
A61K33/20, A61K33/08, A61P1/00, A61P11/00, A61P15/00, A61P17/00,
A61P27/00, A61P31/04, A61P31/10, A61P31/12

Documentation searched other than minimum documentation to the extent that such documents are included in the fields searched
Jitsuyo Shinan Koho          1922-1996   Jitsuyo Shinan Toroku Koho   1996-2008
Kokai Jitsuyo Shinan Koho   1971-2008   Toroku Jitsuyo Shinan Koho   1994-2008

Electronic data base consulted during the international search (name of data base and, where practicable, search terms used)
BIOSIS(STN), CAplus(STN), EMBASE(STN), MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| X | WO 2003/082304 A1 (KLING, William, O), 09 October, 2003 (09.10.03), Examples; page 4, lines 14 to 17 & US 2005/0142215 A1 | 1,7 |
| X | JP 62-106022 A (Kabushiki Kaisha Nasa), 16 May, 1987 (16.05.87), Claims; examples (Family: none) | 1,7 |
| X | JP 4-046003 A (The Japan Carlit Co., Ltd.), 17 February, 1992 (17.02.92), Examples (Family: none) | 1-6 |
| Y | | 1-5,8-11 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 April, 2008 (24.04.08) | 13 May, 2008 (13.05.08) |

| Name and mailing address of the ISA/ Japanese Patent Office | Authorized officer |
|---|---|
| Facsimile No. | Telephone No. |

Form PCT/ISA/210 (second sheet) (April 2007)

<div align="center">26</div>

**INTERNATIONAL SEARCH REPORT**

| International application No. |
|---|
| PCT/JP2008/055384 |

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | JP 2000-063217 A  (Kabushiki Kaisha Di Emu Shi Nettowaku),<br>29 February, 2000 (29.02.00),<br>Par. No. [0012]; examples<br>(Family: none) | 1-6<br>1-5,8-11 |
| X<br>Y | JP 11-278808 A  (Bijinesu Puran Kabushiki Kaisha),<br>12 October, 1999 (12.10.99),<br>Claims; examples; Par. Nos. [0071], [0073]<br>(Family: none) | 1-6<br>1-5,8-11 |
| Y | JP 3-505203 A  (Alcide Corp.),<br>14 November, 1991 (14.11.91),<br>Full text<br>& US 4956184 A          & US 5384134 A<br>& WO 89/010747 A1 | 1-5,8-11 |
| Y | CROUGHAN, W.S. et al, Comparative study of inactivation of herpes simplex virus types 1 and 2 by commonly used antiseptic agents, Journal of clinical microbiology, 1988, Vol.26, No.2, p.213-5 | 1-5,8-11 |
| Y | THURSTON-ENRIQUEZ, J.A. et al, Inactivation of enteric adenovirus and feline calicivirus by chlorine dioxide, Applied and Environmental Microbiology, 2005, Vol.71, No.6, p.3100-3105 | 1-5,8-11 |
| Y | HUANG, J. et al, Disinfection effect of chlorine dioxide on viruses, algae and animal planktons in water, Water Research, 1997, Vol.31, No.3, p.455-460 | 1-5,8-11 |
| A | US 6287551 B1  (Perry A. RATCLIFF),<br>11 September, 2001 (11.09.01),<br>& US 6200557 B1          & US 6017554 A<br>& US 5489435 A          & US 5618550 A<br>& US 5811115 A          & US 6274132 B1<br>& US 6277363 B1          & US 6280716 B1<br>& US 5834003 A          & US 5902575 A<br>& US 5935592 A          & WO 95/001774 A1 | 1-11 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005525090 PCT

- JP 61040803 A